# EUROPÄISCHE PATENTSCHRIFT

(11) **EP 3 700 461 B1**
(45) Veröffentlichungstag und Bekanntmachung des Hinweises auf die Patenterteilung: **20.10.2021**
(21) Anmeldenummer: 20700344.3
(22) Anmeldetag: 07.01.2020
(51) Int. Cl.: A61C 17/16, A61C 19/04, A61B 5/00, A46B 15/00

(54) **HANDGERÄT ZUR FLUORESZENZ-ANREGUNG UND ZUR BESTRAHLUNG VON MIKROORGANISMEN IM MUND- UND RACHENRAUM**
HAND-HELD DEVICE FOR FLUORESCENCE EXCITATION AND FOR IRRADIATING MICROORGANISMS IN THE MOUTH AND THROAT
APPAREIL PORTATIF PERMETTANT L'EXCITATION DE FLUORESCENCE ET L'IRRADIATION DE MICRO-ORGANISMES DANS LA BOUCHE ET LA GORGE

(30) Priorität: 08.01.2019 DE 102019100295
(43) Veröffentlichungstag der Anmeldung: 02.09.2020
(73) Patentinhaber: König, Karsten, 12559 Berlin (DE)
(72) Erfinder: König, Karsten, 12559 Berlin (DE)
(74) Vertreter: Lahrtz, Fritz
(86) Internationale Anmeldenummer: PCT/EP2020/050222
(87) Internationale Veröffentlichungsnummer: WO 2020/144187

(56) Entgegenhaltungen:
- US-A1- 2004 193 235
- US-A1- 2007 198 004
- US-A1- 2008 060 148

## Beschreibung

Die Erfindung betrifft ein Handgerät, beispielsweise in Form einer Zahnbürste, zur Fluoreszenz-Anregung und Bestrahlung von pathogenen Mikroorganismen im Mund- und Rachenraum mittels optischer Strahlung, die dazu geeignet ist, das Auftreten dieser Mikroorganismen, wie zum Beispiel Bakterien, die eine entscheidende Rolle in der Pathogenese von Zahnkaries, Gingivitis und Parodontitis, aber auch bei entzündlichen Veränderungen im Rachenraum, wie zum Beispiel bei einer Sinusitis, spielen, für einen Anwender erkennbar zu machen und die Verbreitung dieser Mikroorganismen durch deren Inaktivierung zu verlangsamen beziehungsweise vollständig zu verhindern, sowie die bereits vorhandenen Mikroorganismen abzutöten.

Genauer gesagt betrifft die Erfindung ein Handgerät zur Fluoreszenz-Anregung und Bestrahlung von pathogenen Mikroorganismen im Mund- und Rachenraum, mit den Merkmalen von Anspruch 1, wie zum Beispiel eine Zahnbürste, mit zumindest einer Anregungslichtquelle im kurzwelligen sichtbaren Spektralbereich zur Eigenfluoreszenzanregung und Bestrahlung der pathogenen Mikroorganismen an der Oberfläche des zu anvisierten Bereichs im Mund- und Rachenraum, wobei die Anregungslichtquelle eine Anregungsstrahlung abgibt, welche einem Absorptionsmaximum von Porphyrinen entspricht, zumindest einer primären Bestrahlungslichtquelle im roten Spektralbereich zur primären Bestrahlung der pathogenen Mikroorganismen und zur Transillumination, und optional zumindest einer sekundären Bestrahlungslichtquelle im sichtbaren Spektralbereich zwischen 450 nm und 600 nm zur sekundären Bestrahlung der pathogenen Mikroorganismen, wobei die Bestrahlungslichtquellen spektrale Anteile aufweisen, welche von endogenen Porphyrinen, die durch die pathogenen Mikroorganismen produziert werden, absorbierbar sind, wodurch anhand von Folgeprozessen eine Fluoreszenzanregung und eine Inaktivierung der pathogenen Mikroorganismen eintritt. Um die unbeabsichtigte Bestrahlung der Augen zu vermeiden, weist das Handgerät zudem einen Drucksensor auf, um höhere Lichtintensitäten erst bei Messung eines Andruckes freigeben, wobei der Drucksensor bei Erfassung eines Andruckes des Handgeräts auf dem betroffenen Oberflächenbereich eine Bestrahlungslichtintensität auf Werte in einem Bereich von 10 mW/cm² bis 100 mW/cm² erhöht, um eine Inaktivierung der pathogenen Mikroorganismen zu erzielen. Zudem ist das Handgerät so eingerichtet, dass die Strahlung das Handgerät divergent verlässt. Die räumlich aufgelöste Detektion der Fluoreszenz der pathogenen Mikroorganismen kann auch genutzt werden, um die Inaktivierung der Bakterien durch gezielte Bestrahlung in dem fluoreszierenden Bereich zu induzieren.

Auf dem Fachgebiet der Zahn-, Mund- und Kieferheilkunde kommen bereits seit geraumer Zeit verschiedenste Techniken und Geräte zum Einsatz, um einen Gesundheitszustand von Zähnen eines Patienten, beispielsweise einen Bakterienbefall direkt auf belagsfreien Zahnoberflächen oder auch Bakterienvorkommen in unterschiedlich tiefen Plaque-Schichten, durch visuelle Untersuchungen oder durch Verwendung von Röntgenstrahlung zu erfassen und darauf beruhend entsprechende Gegenmaßnahmen zu ergreifen. Durch einen derartigen Bakterienbefall im Rachenraum können durch deren Übertragung in die Blutbahn ernsthafte systemische Erkrankungen verursacht werden. Beispielsweise haben klinische Studien bei an Parodontitis Erkrankten ein fast doppelt erhöhtes Risiko für Herzerkrankungen und ein fast dreifach erhöhtes Risiko für das Auftreten eines Schlaganfalls ergeben. Entsprechend kommt effektiven Gegenmaßnahmen hohe Bedeutung zu. Bereits bekannte Gegenmaßnahmen umfassen neben der täglichen Dentalhygiene mittels Zahnseide, Zähneputzen und Mundspülung unter anderem die von einem Fachmann, beispielsweise einem Zahnarzt, durchgeführte Zahnreinigung, wobei auch unter anderem Ultraschall-Reinigungsverfahren zur Anwendung kommen können. Üblicherweise wird dabei jedoch nur eine bedingt ausreichende Zahnbelagsentfernung erzielt. Beispielsweise wird mit der herkömmlichen mechanischen Zahnreinigung durch die Verwendung einer Zahnbürste nur ein Drittel der Zahnoberfläche erreicht. Bei besonders hartnäckigen Fällen kommen oft auch antibiotisch wirkende Pharmaka als bakterieninaktivierende Maßnahme zur Anwendung. Mit der gegenwärtigen Zunahme antibiotikaresistenter Bakterienstämme gewinnt jedoch die Suche nach effizienteren Diagnose- und Behandlungsmöglichkeiten für mikrobielle Infektionen der Zähne an wesentlicher Bedeutung.

In den vergangenen Jahren wurde die antibakterielle Wirkung einer Bestrahlung der Zahnoberflächen mit Laserlicht hoher Intensität bereits genauer untersucht. Dieser Prozess der Schädigung der Bakterien unterliegt dabei im Wesentlichen einem photothermischen Effekt, wodurch zusätzlich durch die ablative Wirkung des Lasers eine abtragende Bakterienentfernung erreicht werden kann. Die offensichtlichen Nachteile dieser Art von Behandlung sind die dazu notwendigen, meist aufwendigen und großen Lasersysteme, sowie die erforderlichen hohen Lichtintensitäten im Bereich von mehreren Kilowatt pro Quadratzentimeter (kW/cm²) bis über ein Gigawatt pro Quadratzentimeter (GW/cm²). Eine andere Behandlungsform zur Entfernung von pathogenen Mikroorganismen findet sich in deren Inaktivierung durch Licht-induzierte chemische Reaktionen unter Beteiligung von lichtsensitiven Stoffen, die den Mikroorganismen in der Regel von außen zugegeben werden müssen. Die vorhergenannten Arten der Therapie erfordern jedoch nicht nur geschultes Fachpersonal und das nötige klinische Equipment, sondern sind darüber hinaus für die tägliche Heimanwendung ungeeignet.

Durch die aufkommende Verwendung lasergestützter Autofluoreszenzspektroskopie bei klinischen Diagnosen wurde in letzter Zeit immer mehr und mehr erkannt, dass verschiedenste Bakterienarten fluoreszierende Stoffe natürlich synthetisieren und bei geeigneter Anregung einen ausgeprägten Photoeffekt durch Autofluoreszenz liefern, der zur Diagnose eines Bakterienbefalls von Organen und Geweben genutzt werden kann. Beruhend auf diesen Erkenntnissen der klinischen Praxis entstand für bei der Entwicklung der vorliegenden Erfindung das Bedürfnis, eine entsprechende Anwendung auf dem medizintechnischen Gebiet der Zahn-, Mund- und Kieferheilkunde zu finden und entsprechende neue medizintechnische Gerätschaften zu entwickeln, die in der Lage sind, einerseits den Gesundheitszustand von Zähnen, insbesondere das Vorhandensein von Karies, Plaque oder bakteriellem Befall an Zähnen, feststellen zu können, sowie bei positivem Feststellen eines Befalls andererseits eine entsprechende Behandlung auf einfache Art und Weise ohne die Nachteile der klinischen Praxis durchführen zu können, d.h. ohne die Notwendigkeit von Fachpersonal oder von aufwendigen und großen Lasersysteme für entsprechende hohe Lichtintensitäten.

Es wurden daher beispielsweise in der Veröffentlichung DE 30 31 249 C2 oder auch in der Veröffentlichung DE 93 17 984 U1 berührungslose Diagnoseverfahren zum Feststellen von Karies, Plaque oder bakteriellem Befall an Zähnen vorgeschlagen, bei denen ein Zahn mit einer nahezu monochromatischen Lichtquelle bestrahlt wird. Aufgrund der Bestrahlung des Zahnes mit monochromatischem Licht wird an dem Zahn eine Fluoreszenzstrahlung angeregt, wobei das Fluoreszenzspektrum deutliche Unterschiede zwischen gesunden und kranken Zahnbereichen aufweist. Durch die Erfassung und Auswertung des Fluoreszenzspektrums des auf diese Weise bestrahlten Zahnes kann somit eindeutig ein gesunder Zahnbereich von einem kranken Zahnbereich unterschieden werden.

Ebenfalls verbreitet ist die Inaktivierung von Mikroorganismen mittels ultravioletter (UV) Strahlung, beispielsweise beschrieben in der Veröffentlichung EP 0 818 181 A1, bei der der Einsatz von UV-Laserstrahlung für den Kariesabtrag vorgeschlagen wird. Nachteile der UV-Strahlung sind neben der geringen Eindringtiefe der Strahlung von wenigen Mikrometern insbesondere das Potential von UV-Strahlung, krebsauslösende Prozesse induzieren zu können.

Weiterhin bekannt auf dem Fachgebiet der Zahn-, Mund- und Kieferheilkunde ist die Inaktivierung von Bakterien mittels sichtbarer Strahlung unter Verwendung bestimmter exogener lichtsensitiver Stoffe, sogenannter Photosensibilisatoren. Bei geeigneter Lichtanregung derartiger Photosensibilisatoren bilden sich infolge von Photooxidationsprozessen Sauerstoffradikale und elektronisch angeregter Sauerstoff, sogenannter Singulett-Sauerstoff, welche in Folgereaktionen mit biologischer Materie zellzerstörend wirken. Die Photosensibilisatoren werden im Zuge der sogenannten photodynamischen Therapie (PDT) insbesondere zur Therapie von Tumoren eingesetzt ((z. B. Cancer Res. 38(1978) S. 2628-2635). In der einschlägigen Literatur (z.B. J. Photochem. Photobiol.B 21(1993) S. 81-86) wurde berichtet, dass die Zugabe von Photosensibilisatoren zu Bakterienkulturen und nachfolgende Lichtaktivierung zu einer Inaktivierung der Bakterien führen kann. Die beschriebene photodynamische Therapie von Geweben und die photodynamische Inaktivierung von Mikroorganismen haben jedoch den entscheidenden Nachteil, dass eben eine Zugabe von Photosensibilisatoren notwendig ist. Um hierbei bei deren Bestrahlung eine hohe Schädigung umgebenden Gewebes zu vermeiden, ist eine Anreicherung der Photosensibilistoren im Target erforderlich. Dies ist im Allgemeinen jedoch nur unzureichend umsetzbar und Gegenstand umfangreicher Forschungsaktivität. Bei bisher klinisch verwendeten exogenen Photosensibilisatoren besteht durch Anreicherung außerhalb des Targetmaterials, z.B. in der gesunden Haut, die Gefahr der hervorgerufenen zeitweisen Lichtempfindlichkeit des Patienten und der unerwünschten Photoschädigung von gesundem Gewebe.

Als ein Beispiel für die Ergebnisse der vorhergehend genannten Forschungsaktivität kann der Veröffentlichung EP 0 743 029 A2 eine Laseranordnung in Verbindung mit Lichtleitern entnommen werden, die auf der photodynamischen Inaktivierung von pathogenen Mikroorganismen im Mund- und Rachenraum unter Verwendung exogener Photosensibilisatoren beruht, wobei die Applikation des Photosensibilisators durch dessen Zugabe in eine Zahnpasta oder in eine Flüssigkeit erfolgen soll. Nachteile dieser Anordnung sind neben dem Einsatz exogener Photosensibilisatoren die Verwendung von Laserlichtleitenden Systemen und die entsprechend erforderlichen hohen Sicherheitsanforderungen durch den Einsatz von Laserstrahlung im Gesichtsbereich. Zudem ist mit der Verabreichung einer den Photosensibilisator enthaltenen Zahnpasta oder Flüssigkeit auch die Gefahr der Anreicherung des Photosensibilisators in der gesunden Schleimhaut gegeben. Eine Bestrahlung der mit dem Photosensibilisator angereicherten Schleimhaut insbesondere im Zahnbereich und der Zunge kann wesentliche Gewebeschädigungen hervorrufen.

Ferner ist bekannt, dass entsprechende Bakterien im Rachenraum ebenfalls zu Entzündungszuständen führen können, so zum Beispiel bei einer Sinusitis, auch als Nasennebenhöhlenentzündung bezeichnet, einschließlich einer Sinusitis maxillaris, auch als Kieferhöhlenentzündung bezeichnet, die durch Viren- und Bakterienbefall bei einem hohen Prozentsatz der europäischen Bevölkerung regelmäßig auftritt. So sind zum Beispiel in Deutschland jährlich ca. 15% der Einwohner von diesen Krankheitszuständen betroffen. Durch Schwellung der Schleimhäute kann dabei Sekret nicht mehr ordnungsgemäß abfließen. Besteht der Krankheitszustand mehr als eine Woche, sind üblicherweise Bakterien involviert. Besteht der Zustand mehr als zwei Monate, spricht man von einer chronischen Sinusitis. Die Diagnose einer Sinusitis beruht bislang auf der Untersuchung des Nasensekrets, oder aber erfolgt anhand von Computertomographie (CT) oder Magnetresonanztomographie (MRT), welche jedoch teure und aufwendige Verfahren darstellen und insbesondere für Kinder und Jugendliche wegen der Strahlenbelastung wenig geeignet sind. Optisch können Untersuchungen des Rachenraums jedoch auch endoskopisch durchgeführt werden. Eine weitere optische Methode wurde durch Wang et al. 2005 publiziert, wobei ein nahe infrarote (NIR-)Lichtquelle im Rachenraum und eine NIR-empfindliche CCD-Kamera zum Einsatz kommt, die die transilluminierte NIR-Strahlung detektiert. Flüssigkeitsansammlungen infolge Sinusitis führen zu einer veränderten Transillumination (Wang et al. "Near infrared transillumination of the maxillary sinuses: Overview of methods and preliminary clinical results." Proceed. SPIE 5686 (2005)). Nachteil dieser Anordnung ist der Einsatz von nicht-sichtbaren NIR-Lichtquellen und spezieller NIR-sensitiver CCD-Kameras, wobei zu beachten ist, dass die meisten CCD-Kameras mit NIR-Blockierungsfiltern ausgestattet sind, die eine Detektion der NIR-Strahlung verhindern.

Aufgabe der vorliegenden Erfindung ist entsprechend die Bereitstellung eines einfach in Heimanwendung zu handhabenden und für einen Anwender ungefährlichen Handgeräts in Gestalt einer Zahnbürste bzw. einer sogenannten Lichtzahnbürste, zur Transillumination sowie zur photodynamischen Inaktivierung von pathogenen Mikroorganismen im Mund- und Rachenraum, welches nicht auf den Einsatz von exogenen Photosensibilisatoren angewiesen ist, wie zum Beispiel aus den Veröffentlichungen US 2008/060148 A1, US 2004/193235 A1 oder US 2007/198004 A1 bekannt. Hierbei ist unter anderem eine hohe Eindringtiefe der Strahlung in das betroffene Gewebe sowie eine Dosierbarkeit der Strahlungsintensität von Vorteil.

Aus diesbezüglicher eigener Forschung bei der Entwicklung der vorliegenden Erfindung wurde gefunden, dass bestimmte pathogene Mikroorganismen, welche insbesondere im Mund- und Rachenraum auftreten, unter anderem die metallfreien, fluoreszierenden und lichtsensitiven Porphyrine Protoporphyrin IX (PP EX) und Coproporphyrin (CP) natürlich synthetisieren (Cell Mol. Biol. 46(2000) S. 1297-1303). Derartige Mikroorganismen umfassen beispielsweise die die pathogenen Gram-positiven Porphyrin produzierenden ATCC-Stämme Propionibacterium acnes, Actinomyces odontolyticus und Porphyromonas gingivalis sowie die Prevotella Spezies, welche bei der Pathogenese und Ausprägung von Karies, Ginigvitis und Paradontitis eine wesentliche Rolle spielen können, und welche die genannten Porphyrine ohne künstliche Stimulation von Außen synthetisieren. Entsprechend sind diese Mikroorganismen als Träger endogener Photosensibilisatoren sensibel gegenüber sichtbarem Licht. Bei weiteren diesbezüglichen Untersuchungen wurde anhand von Fluoreszenzuntersuchungen gefunden, dass sich Protoporphyrin IX und Coproporphyrin in kariösen Zähnen sowie in dentaler Plaque (Zahnbelag) ansammeln (Cell Mol. Biol. 44(1998) S. 1293-1300).

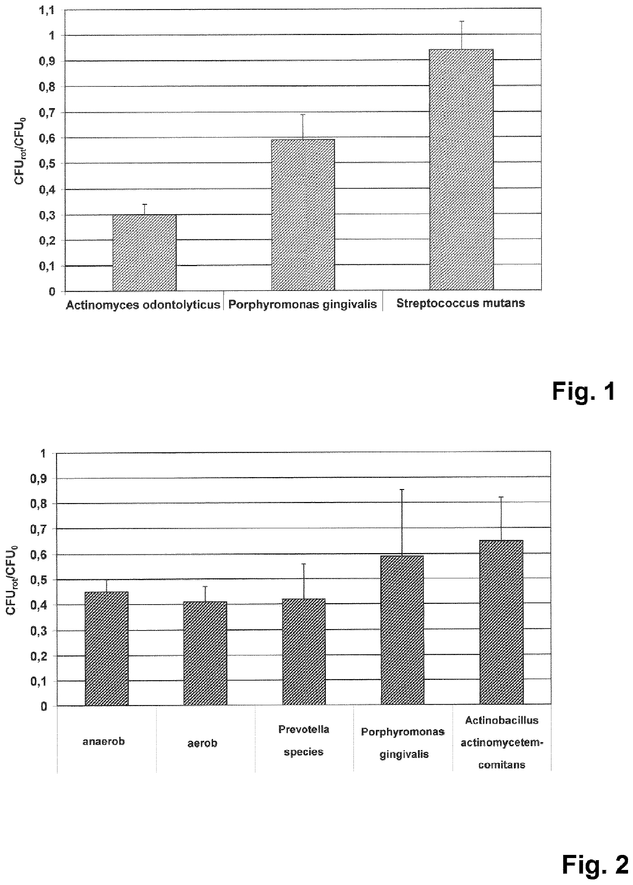

Überraschenderweise stellte sich bei den Untersuchungen während der Entwicklung der vorliegenden Erfindung heraus, dass eine Inaktivierung der vorhergehend genannten pathogenen Gram-positiven Porphyrin produzierenden ATCC-Stämme durch Bestrahlung im roten Spektralbereich um 633 nm und sehr geringen und damit für Gewebe ungefährlichen Bestrahlungsintensitäten im Bereich von Milliwatt pro Quadratzentimeter (mW/cm²) möglich ist, ohne dass ein exogener Photosensibilisator verwendet werden muss. Beispielhafte Ergebnisse dieser eigenen Forschungen können Figuren 1 und 2 entnommen werden, in denen der Quotient aus Anzahl koloniebildender Einheiten (CFU; "colony forming unit") bestrahlter Proben ("CFUᵣₒₜ") zu nichtbestrahlten Proben ("CFU₀") darstellen, wobei dieser Quotient der Überlebensrate entspricht. Kulturen von industriell gezüchteten Bakterien sowie mikrobiologische Proben von Parodontitis-Patienten wurden für diese Experimente einmalig mit Strahlung der Wellenlänge von in etwa 633 nm, beispielsweise 632,8 nm, bei einer Intensität von 100 mW/cm² und einer Energiedichte von 360 J/cm² bestrahlt, beispielsweise mit einem Helium-Neon-Laser. Es konnte dabei ein deutlicher Inaktivierungseffekt bereits bei nur einer einmaligen Bestrahlung beobachtet werden. Wie es Figuren 1 und 2 entnommen werden kann, betrug die mittlere Überlebensrate bei den Porphyrin-haltigen Bakterienstämmen Actinomyces odoniolyticus und Porphyromonas gingivivalis nur 30 % ± 4 % bzw. 59 % ± 10 %. Der Bakterienstamm Streptococcus mutans, bei dem keine nachweisbaren Porphyrine detektiert werden konnten, zeigte dagegen jedoch keine signifikante Inaktivierung. Die Überlebensrate bei mikrobiologische Proben von Parodontitispatienten betrug 45 % bei anaeroben Keimen, 41 % bei aeroben Keimen, 42% bei Prevotella Spezies, 59 % bei Porphyromonas gingivalis und 65 % bei Äctinobacillus actinimycetemcomitans. Diese Ergebnisse zeigen deutlich, dass eine Bestrahlung Porphyrin-haltiger pathogener Mikroorganismen mit Strahlung im roten Spektralbereich ohne den Einsatz exogener Photosensibilisatoren zu einer effektiven Inaktivierung dieser Bakterien führt.

Typischerweise erfolgt die Aktivierung der Photosensibilisatoren entsprechend mit Strahlung im roten Spektralbereich, d.h. im Wellenlängenbereich von 630 bis 700 nm. Derartige Strahlung im roten Spektralbereich liegt im Bereich des sogenannten "optischen Fensters", also dem Bereich relativ hoher Eindringtiefe der Strahlung in biologisches Gewebe. Die Eindringtiefe, die als diejenige Gewebetiefe definiert werden kann, wo der Abfall der Lichtintensität auf in etwa 37% erfolgt ist, liegt typischerweise bei einem bis fünf Millimetern in biologischen Geweben. Eine ebenfalls hohe Eindringtiefe kann durch Strahlung im gelben Spektralbereich erreicht werden, d.h. im Wellenlängenbereich von 560 bis 590 nm. Die Eindringtiefe ultravioletter, violetter und infraroter Strahlung größer 2 µm liegt demgegenüber in biologischen Geweben dagegen typischerweise nur im Mikrometerbereich.

Erfindungsgemäß wird entsprechend ein Handgerät zur Anregung und Bestrahlung von pathogenen Mikroorganismen im Mund- und Rachenraum bereitgestellt, welches zumindest eine Anregungslichtquelle im kurzwelligen sichtbaren Spektralbereich zur Eigenfluoreszenzanregung und Bestrahlung der pathogenen Mikroorganismen an der Oberfläche des zu bestrahlenden Bereichs aufweist, wobei die Anregungslichtquelle (31; 31') eine Anregungsstrahlung abgibt, welche einem Absorptionsmaximum von Porphyrinen entspricht. Der hier genannte kurzwellige sichtbare Spektralbereich umfasst dabei vorzugsweise violettes Licht in einem Bereich von 400 nm bis 410 nm. Typische Lichteindringtiefen in diesem Spektralbereich sind im Zahn wenige hundert Mikrometer, d.h. < 1 mm. Ferner weist das erfindungsgemäße Handgerät zumindest eine primäre Bestrahlungslichtquelle im roten Spektralbereich, d.h. mit einer Wellenlänge um 630 nm, zur primären Bestrahlung der pathogenen Mikroorganismen und zur Transillumination auf, also mit einer dualen Verwendungsmöglichkeit. Ferner weist das erfindungsgemäße Handgerät optional zumindest eine sekundäre Bestrahlungslichtquelle im sichtbaren, vorzugsweise grünen oder gelben, Spektralbereich von 450 nm bis 600 nm zur Bestrahlung der pathogenen Mikroorganismen auf. Die Anregungslichtquelle, als auch die primäre und die optionale sekundäre Bestrahlungslichtquelle geben entsprechend sichtbare Strahlung ab, wobei alle Bestrahlungslichtquellen unterschiedliche Wellenlängen aufweisen, und die abgegebene Strahlung jeweils spektrale Anteile aufweist, welche von endogenen Porphyrinen, die durch die pathogenen Mikroorganismen produziert werden, absorbierbar sind. Dadurch kann anhand von Folgeprozessen eine Fluoreszenzbildung und eine Inaktivierung der pathogenen Mikroorganismen erreicht werden. Zudem ist die Strahlung der primären Bestrahlungslichtquelle im roten Spektralbereich um 630 nm neben der direkten Bestrahlung der Mikroorganismen zu deren Inaktivierung auch dazu geeignet, durch Transillumination entzündliche Prozesse im Rachenraum (Sinusitis) zu erkennen. In anderen Worten weist das erfindungsgemäße Handgerät mindestens eine violette Strahlungsquelle (405 nm) und eine rote Strahlungsquelle (633nm) auf. Die violette Strahlung dient dabei zur Fluoreszenzanregung und Inaktivierung von Bakterien an der Oberfläche des Zahnhartgewebes und des Weichgewebes. Die rote Strahlung der primären Bestrahlungslichtquelle dient im Sinne der bereits genannten dualen Verwendungsmöglichkeit einerseits zur Erkennung von Sinusitis durch veränderte Streustrahlung, die durch Transillumination durch die Haut im Gesichtsbereich detektiert werden kann, also durch diejenigen Strahlungsanteile, die durch eine im Inneren der Mundhöhle eingebrachte Strahlungsquelle generiert werden und die Haut transmittieren und somit im Gesichtsbereich detektiert werden können. Dabei weist das Transillumination-Bild des krankhaften Areals ein räumlich verändertes und Intensitätsverändertes Muster gegenüber gesunden Arealen auf. Andererseits dient die rote Strahlung zudem der Inaktivierung von Bakterien in der Gewebetiefe, d.h. im tieferliegenden Gewebe von mehreren Millimetern, wie zum Beispiel in tieferen Schichten des Zahnhartgewebes. Das erfindungsgemäße Handgerät weist darüber hinaus einen Drucksensor auf, dessen Messergebnis dazu dient, bei Erfassung eines Anpressdruckes des Handgeräts auf dem anvisierten Oberflächenbereich eine Bestrahlungslichtintensität der Bestrahlungslichtquellen auf Werte in einem Bereich von 10 mW/cm² bis 100 mW/cm² zu erhöhen, um eine Intensivierung der Inaktivierung der pathogenen Mikroorganismen zu erzielen. Dadurch kann sichergestellt werden, dass keine zu hohe Bestrahlungsintensität durch das erfindungsgemäße Handgerät bereitgestellt wird, wenn sich das Handgerät nicht im Mund- und Rachenraum befindet, um insbesondere die Bestrahlung der Augen eines Anwenders mit der erhöhten Bestrahlungsintensität zu verhindern.

Die pathogenen Mikroorgansimen entsprechend beispielsweise den die pathogenen Gram-positiven Porphyrin produzierenden ATCC-Stämmen Propionibacterium acnes, Actinomyces odontolyticus und Porphyromonas gingivalis sowie die Prevotella Spezies. Anhand der Anregungslichtquelle ist es bei dem vorliegenden erfindungsgemäßen Handgerät möglich, eine Eigenfluoreszenz dieser pathogenen Mikroorganismen anzuregen, so dass ein Anwender mit bloßem Auge erkennen kann, wo bzw. auf welchen Zähnen oder Zahnbereichen derartige pathogene Mikroorganismen sitzen. Zudem kann durch die Anregungslichtquelle im kurzwelligen sichtbaren Spektralbereich bereits eine erste Inaktivierung der pathogenen Mikroorganismen erfolgen. Anschließend wird es dem Anwender neben der bereits beschriebenen Transillumination ermöglicht, insbesondere durch Verwendung der primären und/oder der optionalen sekundären Bestrahlungslichtquelle die eigenfluoreszierenden und andere pathogenen Mikroorganismen mit Wellenlängen zu bestrahlen, die von den endogenen Porphyrinen, die durch die pathogenen Mikroorganismen produziert werden, absorbiert werden. Durch entsprechende Folgeprozesse, die durch die Absorbierung dieser Strahlung ausgelöst werden, können die Mikroorganismen zur weiteren Fluoreszenzbildung als auch dazu gebracht werden, zumindest teilweise, vorzugsweise vollständig inaktiviert zu werden. Das erfindungsgemäße Handgerät ist folglich geeignet, das Auftreten und die Verbreitung bestimmter pathogener Mikroorganismen zu hemmen und durch Auslösung photodynamischer Reaktionen unter Beteiligung endogener Porphyrine bereits vorhandene derartige Mikroorganismen zu zerstören. Die Gabe eines exogenen Photosensibilisators ist dazu nicht weiter erforderlich.

Gemäß einer bevorzugten Ausführung gibt die Anregungslichtquelle zur Eigenfluoreszenzanregung der pathogenen Mikroorganismen eine Anregungsstrahlung ab, die einem Absorptionsmaximum von Porphyrinen entspricht, wobei die Anregungsstrahlung beispielsweise in einem Bereich von 400 nm bis 410 nm liegen kann, wie zum Beispiel im Bereich von 405 nm, was violettem Licht entspricht. Dadurch kann eine möglichst maximale Eigenfluoreszenz der anvisierten Mikroorganismen erreicht werden, um es einem Anwender so einfach wie möglich zu machen, möglichst alle Mikroorganismuskolonien auf dessen Zahnoberflächen zu entdecken, um diese zielgerichtet bekämpfen zu können. Die Strahlung erreicht dabei jedoch üblicherweise noch keine tiefe Eindringtiefe, so dass damit verstärkt Mikroorganismen an der Oberfläche anvisiert und inaktiviert werden können, aufgrund der Eigenschaft von violettem Licht in Form von dessen geringer Lichteindringtiefe in biologisches Weich- und Hartgewebe.

Gemäß einer weiteren bevorzugten Ausführung der vorliegenden Erfindung wird zudem mit dem erfindungsgemäßen Handgerät Strahlung im längerwelligen sichtbaren Spektralbereich abgegeben. Die Bestrahlung weist dabei beispielsweise einen zusätzlichen Wellenlängenanteil in einem Bereich von 630 nm bis 700 nm auf, vorzugsweise in einem Bereich von 630 nm bis 635 nm, weiter vorzugsweise im Bereich von 633 nm Licht, was rotem Licht entspricht. Bei Anwendung von Strahlung im Bereich von 630 nm bis 640 nm ist eine hohe Eindringtiefe der Strahlung sowie die gezielte Einstrahlung in eine Absorptionsbande der endogenen Porphyrine Protoporphyrin IX und Coproporphyrin gegeben, wodurch tieferliegende Mikroorganismen erreicht und dadurch inaktiviert werden können. Durch die aufeinanderfolgende Bestrahlung mit Strahlung der Anregungslichtquelle und anschließend mit Strahlung der primären Bestrahlungslichtquelle mit einem Wellenlängenanteil in einem Bereich von 630 nm bis 700 nm, vorzugsweise in einem Bereich von 630 nm bis 635 nm, weiter vorzugsweise im Bereich von 633 nm, und optional auch einer Bestrahlung durch die sekundäre Bestrahlungslichtquelle mit einem Wellenlängenanteil in einem Bereich von 490 nm bis 560 nm (grün-gelb), vorzugsweise im Bereich von 505 nm, kann eine Bestrahlung des gewünschten Bereichs mit unterschiedlichen Eindringtiefen erreicht werden, um eine möglichst umfängliche Inaktivierung der anvisierten Mikroorganismen zu erreichen. Optional kann also eine dritte Strahlungsquelle im sichtbaren Bereich, vorzugsweise mit grünem oder gelbem Licht, genutzt werden, die bevorzugt der Inaktivierung oberflächiger und etwas tiefer gelegener Bakterien dient. Die Lichtquellen können ferner auch ausschließlich in dem angegebenen Bereich emittieren, kontinuierlich oder gepulst, können aber alternativ auch alternierend Strahlung in einem weiteren sichtbaren Wellenlängenbereich abstrahlen, sowie eine Weißlichtquelle darstellen, falls gewünscht. Krebsauslösende Wirkungen durch Strahlung in den genannten Bestrahlungswellenlängenbereichen sind nicht bekannt. Kurzwellige sichtbare Strahlung im blauen bzw. violetten Spektralbereich, insbesondere um 405 nm, und im gelben oder grünen Spektralbereich, insbesondere um 505 nm, kann eingesetzt werden, da die endogenen Porphyrine hier Absorptionsbanden besitzen. Da die Einstrahltiefe jedoch geringer als im roten Spektralbereich ist, können durch die kurzwellige sichtbare Strahlung nur vorwiegend Mikroorganismen im Oberflächenbereich inaktiviert werden. Bei Einsatz von Strahlung in den genannten Wellenlängenbereichen ist die Applikation von exogenen Photosensibilisatoren zur Inaktivierung von Porphyrin-produzierenden pathogenen Mikroorganismen nicht erforderlich, und das erfindungsgemäße Handgerät ist ohne weitere Hilfsmittel, wie eben exogene Photosensibilisatoren, in der Lage, die anvisierten pathogenen Mikroorganismen zu inaktivieren und dadurch potentiell entstehende Gefahren für den Anwender, wie zum Beispiel Karies, Ginigvitis und Paradontitis, zu verhindern. Alle Strahlungsquellen des erfindungsgemäßen Handgeräts enthalten entsprechend spektrale Anteile, die denen der endogen Porphyrine entsprechen und alle können damit Fluoreszenz und photochemische Prozesse zur Inaktivierung auslösen.

Gemäß einer weiteren bevorzugten Ausführung der vorliegenden Erfindung erfolgt das Abgeben der Strahlung der Anregungslichtquelle, der Strahlung der primären Bestrahlungslichtquelle und der Strahlung der sekundären Bestrahlungslichtquelle nach Art und Weise einer Ampelschaltung. Entsprechend wird zuerst die Strahlung der Anregungslichtquelle abgegeben, beispielsweise an einem oberen Ende des Handgeräts. Nach Erkennung der eigenfluoreszierenden Mikroorganismen und Bestrahlung der Mikroorganismen mit der Anregungslichtquelle in einem Oberflächenbereich kann im Anschluss die Strahlung der primären Bestrahlungslichtquelle erfolgen, die beispielsweise unterhalb der Anregungslichtquelle angeordnet ist. Daraufhin kann die Strahlung der optionalen sekundären Bestrahlungslichtquelle erfolgen, die beispielsweise unterhalb der primären Bestrahlungslichtquelle angeordnet sein kann. Demnach findet eine Bestrahlung beispielsweise der Zähne und des umliegenden Weichgewebes eines Anwenders zuerst mit Anregungsstrahlung an einer Position an dem Handgerät statt, danach findet eine Bestrahlung der Mikroorganismen im Mund- und Rachenbereich des Anwenders mit Bestrahlung aus der primären Bestrahlungslichtquelle an einer weiter unten angeordneten Position an dem Handgerät statt, und im Anschluss findet eine Bestrahlung der Mikroorganismen im Mund- und Rachenbereich des Anwenders mit Bestrahlung aus der sekundären Bestrahlungslichtquelle an einer noch weiter unten angeordneten Position an dem Handgerät statt. Entsprechend kann eine derartige Ampelschaltung der verschiedenen Lichtquellen des erfindungsgemäßen Handgeräts nicht nur einen medizintechnischen Zweck, sondern zudem einen ästhetischen Zweck erfüllen, wobei das Durchlaufen der Ampelschaltung ebenfalls einen Steuerungs- und Kontrollierbarkeitsweck im Sinne eines korrekten Ablaufs der Bestrahlung erfüllt.

Gemäß einer weiteren bevorzugten Ausführung der vorliegenden Erfindung kann das Handgerät ferner zumindest eine Lichterfassungseinrichtung zum Erfassen einer Eigenfluoreszenzstrahlung der pathogenen Mikroorganismen aufweisen, so dass der Anwender nicht darauf angewiesen ist, visuell alle eigenfluoreszierenden Mikroorganismen zu erfassen, sondern kann diese Aufgabe der Lichterfassungseinrichtung überlassen. Die Lichterfassungseinrichtung kann entsprechend die Eigenfluoreszenz der angeregten Mikroorganismen erfassen und ein entsprechendes Erfassungssignal ausgeben, beispielsweise anhand einer haptischen oder akustischen Rückmeldung (feedback) an den Anwender, wobei die Stärke der Rückmeldung die Intensität der Eigenfluoreszenz wiedergeben kann. Ferner kann die Lichterfassungseinrichtung das Erfassungssignal, das heißt die Verteilung der fluoreszierenden Mikroorganismen auf den Zähnend es Anwenders anhand einer Sendeeinrichtung nach außen abgeben, so zum Beispiel über einen schnurlose Bluetooth-Verbindung oder dergleichen an einen Computer, ein Mobiltelefon oder dergleichen, mit dessen Hilfe der Anwender die Verteilung der fluoreszierenden Mikroorganismen auf seinen Zähnen erkennen und entsprechende Schritte einleiten kann, insbesondere in Bezug auf die Notwendigkeit der Bestrahlung durch die primäre und/oder die optionale sekundäre Bestrahlungslichtquelle, oder auch in Bezug auf die jeweilige Bestrahlungsdauer. Fluoreszenzstrahlung und transmittierte Strahlung können ferner mit dem Auge, mit CCD-Kameras wie zum Beispiel in Mobiltelefonen vorhanden, und mit anderen Photonendetektoren, wie zum Beispiel mit einer Lichtdiode oder einem Photomultiplier detektiert werden.

Gemäß einer weiteren bevorzugten Ausführung der vorliegenden Erfindung können die Anregungslichtquelle, die primäre Bestrahlungslichtquelle und/oder die optionale sekundäre Bestrahlungslichtquelle zumindest eine Licht-emittierende Diode (LED), eine organische Leuchtdiode (OLED) und/oder einen Laser aufweisen. Entsprechend kann das erfindungsgemäße Handgerät Strahlungsquellen in Gestalt der Anregungslichtquelle, der primären Bestrahlungslichtquelle und/oder der optionalen sekundären Bestrahlungslichtquelle aufweisen, bevorzugt LEDs hoher Divergenz. Beispielsweise können die LEDs in Form eines Arrays an oder in dem Handgerät angeordnet sein. Durch die bevorzugte hohe Divergenz kann vorteilhafterweise erreicht werden, dass ein Anwender bei fälschlicher Anwendung der Bestrahlungslichtquellen des Handgeräts in dessen Augen keine effiziente Fokussierung der Lichtstrahlung auf die Netzhaut des jeweiligen Auges gegeben ist, und entsprechend eine mögliche Schädigung im Augenbereich bei unsachgemäßer Anwendung des Handgeräts unwahrscheinlich ist. Durch den Einsatz von miniaturisierten Lichtquellen, insbesondere LEDs, kann ein Stromverbrauch bei der Bestrahlung gering gehalten werden, und alle Vorteile, die durch LEDs üblicherweise erzielt werden, wie deren Langlebigkeit, etc., gelten ebenfalls für das erfindungsgemäße Handgerät. Zudem kann das Gerät mit einem Drucksensor ausgerüstet sein, durch den eine Umschaltung auf höhere Lichtintensität erst bei Anliegen eines Druckes ausgelöst wird, der dem typischen Druck einer Zahnbürste auf das Zahnmaterial beim Zähneputzen entspricht. Bevorzugt ist der Drucksensor vorteilhafterweise im Zahnbürstenkopf lokalisiert.

Das Handgerät gemäß der vorliegenden Erfindung ist bevorzugt ein Zahnreinigungsgerät zum Zweck der Nutzung während der täglichen Dentalhygiene, wie zum Beispiel eine Handzahnbürste zur manuellen Reinigung der Zähne oder eine automatische bzw. elektrische Zahnbüste zur automatischen Reinigung der Zähne, wobei sich die Anregungslichtquelle, die primäre Bestrahlungslichtquelle und/oder die optionale sekundäre Bestrahlungslichtquelle in einem Bürstenkopf des Zahnreinigungsgeräts befinden können und eine Abstrahlung in Borstenrichtung erfolgt, das heißt in Richtung der Borsten, die sich von dem Handgerät weg erstrecken. Genauer gesagt kann das Handgerät eine manuell betriebene Zahnbürste sein, auch als Lichtzahnbürste bezeichnet, bei der der Bürstenkopf der Zahnbürste mit einem auswechselbarem Borstenträger versehen sein kann, oder aber das Handgerät kann eine elektrisch betriebene Zahnbürste sein, bei der der in Drehung und/oder Schwingung versetzbare Bürstenkopf auswechselbar sein kann, und wobei eine Stromversorgung des elektrischen Antriebs des Bürstenkopfs und eine Stromversorgung der Anregungslichtquelle, der primären Bestrahlungslichtquelle und/oder der sekundären Bestrahlungslichtquelle durch dieselbe Energiequelle erfolgen kann.

Alternativ ist es denkbar, das Handgerät nach Art einer temporär einsetzbaren Zahn-Kunststoffschiene umzusetzen, wie zum Beispiel einer individuell angepassten tiefgezogenen Zahn-Kunststoffschiene (Überzug über Zähne und Kiefer aus lichtdurchlässigem Kunststoff), so zum Beispiel einer Oberkopfschiene oder einer Unterkieferschiene, welche auf die Zähne eines Kiefers des Anwenders aufgesetzt werden können, wobei Stromquelle, Schalter und LED-Strahlungsquellen in den Kunststoffkörper integriert und an beliebigen Stellen des Kunststoffträgers positioniert sein können. Als weitere, jedoch nicht bevorzugte, Alternative ist es ebenso denkbar, das Handgerät zur Anregung und Bestrahlung von pathogenen Mikroorganismen im Rachenraum ohne Putzfunktion umzusetzen.

Generell kann die oben genannte Anregungslichtquelle bei geringer Intensität zur Bakterienerkennung im Rachenraum durch Fluoreszenzaktivierung von Porphyrinen in den pathogenen Mikroorganismen und bei höherer Intensität zur Inaktivierung oberflächlicher pathogener Mikroorganismen dienen. Ferner kann die primäre Bestrahlungslichtquelle zur Inaktivierung tieferliegender pathogener Mikroorganismen oder als Transmissionsquelle zur Transillumination für die Detektion und zur Verlaufsverfolgung von Sinusitis dienen. Darüber hinaus kann die optionale sekundäre Bestrahlungslichtquelle zur weiteren Inaktivierung pathogener Mikroorganismen dienen, sozusagen als ergänzende Bestrahlung in einem nebengeordneten Strahlungsbereich. Entsprechend kann die Anregungslichtquelle violettes Licht bei 405 nm ausstrahlen, die primäre Bestrahlungslichtquelle rotes Licht bei 633 nm, und die sekundäre Bestrahlungslichtquelle gelbes oder grünes Licht bei 505 nm ausstrahlen, wobei das violette Licht und das rote Licht der Mikroorganismenerkennung im Rachenraum dienen können, und das violette Licht zur Fluoreszenzaktivierung von Porphyrinen in den Mikroorganismen dienen kann, also bei geringerer Intensität zur Fluoreszenzanregung und bei höherer Intensität zur Inaktivierung oberflächlicher Mikroorganismen eingesetzt werden kann. Das rote Licht kann zudem durch Transillumination die Detektion von entzündlichen Veränderungen, wie zum Beispiel Sinusitis, und die Verfolgung dessen Verlaufs ermöglichen. Das grüne Licht kann ähnlich wie das rote Licht bei höherer Intensität zur Inaktivierung von in tieferen Gewebeschichten liegenden Mikroorganismen dienen. Höhere Bestrahlungslichtintensitäten in einem Bereich von 10 mW/cm² bis 100 mW/cm² werden jedoch mit der erfindungsgemäßen Lichtzahnbürste erst ausgestrahlt, wenn der Drucksensor einen Anpressdruck erfasst, welcher signalisiert, dass die Lichtzahnbürste zur Zahnreinigung entweder mit der Zahnoberfläche oder mit dem Gewebe im Mund- und Rachenraum in Kontakt getreten ist. Ein derartiger Drucksensor kann in Form eines piezoresistiven oder piezoelektrischen Drucksensors oder auch in Form eines Hall-Effekt-Drucksensors vorliegen, wobei jede auf dem Markt übliche Art eines Drucksensors verwendet werden kann, der aufgrund seiner Baugröße und/oder Wirkungsweise in der erfindungsgemäßen Lichtzahnbürste verbaut werden kann. Der Drucksensor befindet sich vorteilhafterweise im Zahnbürstenkopf gegenüber der Borsten.

Erfindungsgemäß soll die rote Strahlung um 630 nm des erfindungsgemäßen Handgeräts zudem zur Transillumination verwendet werden. Die rote Strahlung bei 630 nm liegt hierbei im optischen Fenster von biologischem Gewebe und zeichnet sich durch eine hohe Lichteindringtiefe von mehreren Millimetern aus. Die Lichteindringtiefe beschreibt in der Regel den Wert, bei dem die Lichtintensität auf ca. 37% der Anfangsintensität abgefallen ist. Viele rote Photonen können somit durchaus Strecken von mehreren Zentimetern zurücklegen. Dadurch kann die rote Strahlung auch durch die Haut transmittiert werden. Die rote transilluminierte Strahlung kann leicht mit dem Auge detektiert werden. Für die Bildaufnahme eignet sich zudem beispielsweise auch jedes mit einer normalen CCD-Kamera ausgerüstetes Mobiltelefon, oder auch andere Photonendetektoren, wie zum Beispiel Lichtdioden und Photomultiplier (Sekundärelektronenvervielfacher). Dadurch kann sowohl Sinusitis erkannt als auch deren Verlauf beobachtet und dokumentiert werden. Das erfindungsgemäße Handgerät und deren Verwendung sind besonders für Kinder und Jugendliche geeignet. Blut absorbiert im Bereich um 400 nm (Soret-Bande) und im Bereich 540 nm bis 580 nm. Der endogene Absorber Protoporphyrin absorbiert um 405 nm, bei 505 nm, 540 nm, 573 nm und 635 nm. Um höhere Eindringtiefen als bei 400 nm zu erhalten und von beispielsweise dem Protoporphyrin PP IX absorbiert zu werden, solle erfindungsgemäß Licht im Bereich um 505 nm (grün) und 633 nm (rot) eingesetzt werden. Damit könne auch Bakterien in der Gewebetiefe, insbesondere im Weichgewebe, inaktiviert werden. Die Strahlung um 405 nm dient der oberflächlichen Anregung der Fluoreszenz als auch der Inaktivierung von Bakterien an der Oberfläche.

Gemäß einer bevorzugten Ausführung der vorliegenden Erfindung weist das Handgerät eine integrierte Energiequelle zur Stromversorgung der Anregungslichtquelle, der primären Bestrahlungslichtquelle und/oder der sekundären Bestrahlungslichtquelle auf, wie zum Beispiel eine Batterie oder einen Akkumulator, wobei die integrierte Energiequelle im Falle einer Umsetzung als Akkumulator kabellos aufladbar sein kann.

In der nachfolgenden Beschreibung der bevorzugten Ausführungsbeispiele der Erfindung werden gleiche oder ähnliche Komponenten und Elemente mit gleichen oder ähnlichen Bezugszeichen bezeichnet, wobei auf eine wiederholte Beschreibung dieser Komponenten oder Elemente in Einzelfällen verzichtet wird. Die Figuren stellen den Gegenstand der Erfindung nur schematisch dar.

Die Erfindung wird nachfolgend anhand von den nachfolgend beschriebenen Figuren näher erläutert, wobei:
- Fig.1: die Überlebensrate von verschiedenen industriell gezüchteten Bakterienstämmen nach einmaliger Bestrahlung mit 632,8 nm-Strahlung zeigt;
- Fig. 2: die Überlebensrate von mikrobiologischen Proben von Parodontitis-Patienten nach einmaliger Bestrahlung mit 632,8 nm-Strahlung zeigt;
- Fig. 3: eine schematische Schnittdarstellung einer erfindungsgemäßen Lichtzahnbürste ohne elektrisch gesteuerte Bürstenbewegung für die tägliche mechanische und optische Dentalhygiene zeigt;
- Fig. 4: eine schematische Darstellung eines Bürstenkopfs der erfindungsgemäßen Lichtzahnbürste von Fig. 3 zeigt; und
- Fig. 5: eine schematische Schnittdarstellung einer erfindungsgemäßen Lichtzahnbürste mit elektrisch gesteuerter Bürstenbewegung für die tägliche mechanische und optische Dentalhygiene zeigt.

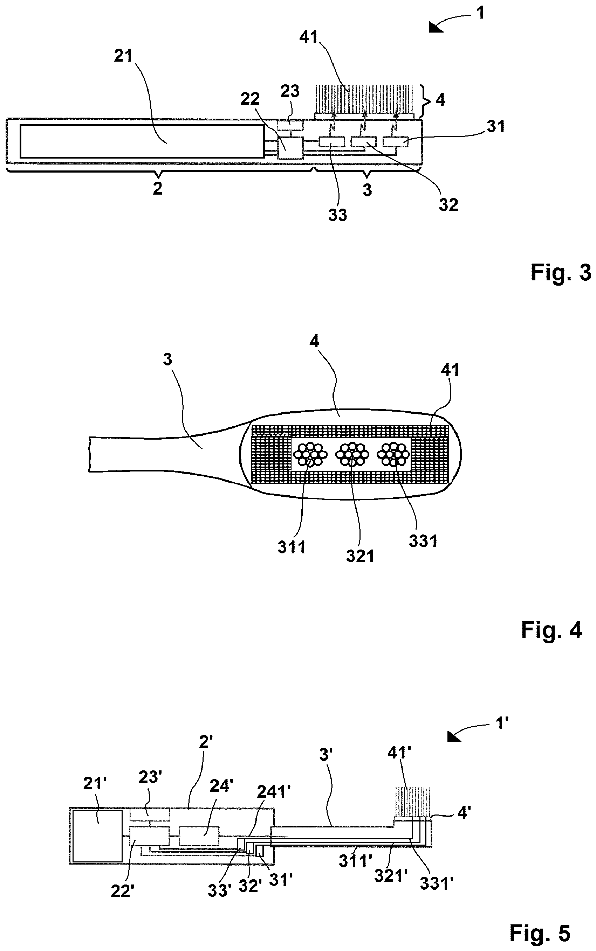

Das erfindungsgemäße Handgerät zur Anregung und Bestrahlung von pathogenen Mikroorganismen im Mund- und Rachenraum ist gemäß einer bevorzugten Ausführung als eine manuell zu betreibende mechanische Zahnbürste 1 zur täglichen mechanischen und optischen Dentalhygiene mittels mechanischer Zahnbelagreduktion und photodynamischer Inaktivierung von Mikroorganismen im Mund- und Rachenraum umgesetzt, wie es beispielhaft in Fig. 3 gezeigt ist. Entsprechend kann die erfindungsgemäße Zahnbürste 1 auch als Lichtzahnbürste 1 bezeichnet werden, da eine Reinigungswirkung nicht nur durch die mechanische Reinigung, sondern zudem durch die Ausstrahlung von Licht erreicht wird. Die Zahnbürste 1 besteht dabei im Wesentlichen aus einem Zahnbürstenschaft 2, einem Zahnbürstenkopf 3 und einem abnehmbarem Borstenträger 4. Eine Strom- bzw. Energiequelle 21 ist in dem Zahnbürstenschaft 2 integriert, beispielsweise in Form eines wiederaufladbaren Akkumulators, der kabellos geladen werden kann, wie zum Beispiel durch nicht drahtgebundene elektromagnetische Felder. Die Energiequelle 21 kann alternativ auch als austauschbare Energiequelle ausgeführt sein, beispielsweise in Gestalt einer auswechselbaren AA-Batterie, oder auch in Form einer miniaturisierten Energiequelle, wie zum Beispiel einer auswechselbaren oder auch wiederaufladbaren Knopfzelle, um das Gewicht der Zahnbürste 1 generell zu verringern. In dem Zahnbürstenschaft 2 ist ferner eine Schaltungslogik 22 angeordnet, beispielsweise in Form eines Computerchips oder dergleichen, der eine Stromverteilung an Lichtquellen 31, 32, 33 regelt, wobei zudem eine Schnittstelle in Gestalt eines Druckknopfs oder Schalters 23 in dem Zahnbürstenschaft 2 vorgesehen ist, durch welchen ein Anwender mit dem Computerchip interagieren kann, beispielsweise um die Ansteuerung der Lichtquellen 31, 32, 33 zu steuern. Der Schalter 23 kann dabei auch in Form eines Touchscreens vorgesehen sein, auf dem eine beispielsweise eine Energieversorgung der einzelnen Lichtquellen 31, 32, 33 angezeigt werden kann. Ferner weist die Lichtzahnbürste 1 einen Drucksensor (nicht gezeigt) auf, der einen Anpressdruck des Borstenträgers 4 bzw. des Zahnbürstenkopfs 3 gegen die anvisierte Oberfläche erfasst und diesen mit dem üblichen Anpressdruck beim Zähneputzen vergleicht, wodurch mittels der Schaltungslogik 22 erkennbar ist, ob sich der Borstenträger 4 bzw. der Zahnbürstenkopf 3 in Anpresskontakt mit einer Zahnoberfläche oder dergleichen im Mund- und Rachenraum befindet. Aus Sicherheitsgründen kann erst bei dieser Erkennung die Schaltungslogik 22 eine Lichtintensität der Lichtquellen 31, 32, 33 ansteuern und diese entsprechend in einen Bereich von 10 mW/cm² bis 100 mW/cm² erhöhen, wodurch ein beispielsweise für Augen gefährlicher Lichtintensitätswert erreicht werden kann.

Die Lichtquellen 31, 32, 33 befinden sich in dem Zahnbürstenkopf 3 der Zahnbürste 1 und umfassen bei dem vorliegenden Ausführungsbeispiel eine Anregungslichtquelle 31, eine primäre Bestrahlungslichtquelle 32 und optional eine sekundäre Bestrahlungslichtquelle 33, die im Betrieb der Zahnbürste 1 in einer Anordnung von oben nach unten, bzw. in der Fig. 3 von rechts nach links angeordnet sind, also in Gestalt einer Ampelanordnung. Jede der Lichtquellen 31, 32, 33 besteht bei dem vorliegenden Ausführungsbeispiel aus einer LED bzw. einer Anordnung von mehreren LEDs, wie zum Beispiel einem sogenannten LED-Array, wobei die LED-Lichtquellen 31, 32, 33 durch Betätigung des Schalters 23 angesteuert werden können. Alternativ dazu kann jede der Lichtquellen auch aus einer OLED bzw. einer Anordnung von OLEDs, oder auch aus einem oder mehreren Lasern bestehen, die durch Betätigung des Schalters 23 angesteuert werden können. Wie es in Fig. 3 durch Zick-Zack-Pfeile angedeutet ist, geben die Lichtquellen 31, 32, 33 Strahlung ab, die durch den Borstenträger 4 sowie dessen Borsten 41 divergent hindurch nach außen abgestrahlt wird. Dies kann ohne Zuhilfenahme weiterer optischer Komponenten umgesetzt werden.

In dem vorliegenden Ausführungsbeispiel wird die Strahlungsübertragung jedoch durch Lichtfasern 311, 321, 331 umgesetzt, wobei ein Lichtfaserbündel 311 die Anregungsstrahlung im Bereich von 400 nm bis 410 nm, vorzugsweise 405 nm, von der Anregungslichtquelle 31 nach außen durch die Borsten 41 hindurch leitet, ein Lichtfaserbündel 321 die Bestrahlungsstrahlung der primären Bestrahlungslichtquelle im Bereich von 630 nm bis 700 nm, vorzugsweise 633 nm von der primären Bestrahlungslichtquelle 32 nach außen durch die Borsten 41 hindurch leitet, und ein optionales Lichtfaserbündel 331 die optionale Bestrahlungsstrahlung der optionalen sekundären Bestrahlungslichtquelle im Bereich von 490 nm bis 560 nm, vorzugsweise 505 nm, von der optionalen sekundären Bestrahlungslichtquelle 33 nach außen durch die Borsten 41 hindurch leitet. Bei der vorliegenden Ausführung sind die emittierenden Enden der Lichtfaserbündel 311, 321, 331 in einem von Borsten 41 freigehaltenen Bereich des Zahnbürstenkopfs 3 angeordnet, wie in Fig. 4 gezeigt. Alternativ können die Borsten 41 jedoch die komplette Fläche des Borstenträgers 4 bedecken, wobei die Borsten 41 selbst als Verlängerung der Lichtfaserbündel 311, 321, 331 dienen können, i.e. als Lichtfasern ausgeführt sein können. Prinzipiell kann das erfindungsgemäße Handgerät auch in Gestalt einer manuell betriebenen mechanischen Zahnbürste mit nicht abnehmbarem Borstenträger umgesetzt werden, oder aber in Gestalt einer elektrisch betriebenen Zahnbürste mit abnehmbaren Zahnbürstenkopf, wobei dann die Lichtquellen in dem festen Zahnbürstenschaft angeordnet sein können, und lediglich eine Strahlungsführung von den Lichtquellen nach außen entkoppelbar vorgesehen sein muss.

Ferner kann in dem vorliegenden Ausführungsbeispiel die Zahnbürste 1 zumindest eine Lichterfassungseinrichtung (nicht gezeigt) zum Erfassen einer Eigenfluoreszenzstrahlung der pathogenen Mikroorganismen angeordnet sein, so dass der Anwender nicht darauf angewiesen ist, visuell alle eigenfluoreszierenden Mikroorganismen zu erfassen, sondern kann diese Aufgabe der Lichterfassungseinrichtung überlassen. Die Lichterfassungseinrichtung kann entsprechend die Eigenfluoreszenz der angeregten Mikroorganismen erfassen und ein entsprechendes Erfassungssignal ausgeben, beispielsweise anhand einer haptischen oder akustischen Rückmeldung (feedback) an den Anwender, wobei die Stärke der Rückmeldung die Intensität der Eigenfluoreszenz wiedergeben kann, oder auch als visuelle Anzeige auf dem als Touchscreen ausgebildeten Schalter 23. Ferner kann die Lichterfassungseinrichtung das Erfassungssignal, das heißt die Verteilung der fluoreszierenden Mikroorganismen auf den Zähnen des Anwenders, anhand einer Sendeeinrichtung (nicht gezeigt) nach außen abgeben, so zum Beispiel über einen schnurlose Bluetooth-Verbindung oder dergleichen an einen Computer, ein Mobiltelefon oder dergleichen, mit dessen Hilfe der Anwender die Verteilung der fluoreszierenden Mikroorganismen auf seinen Zähnen erkennen und entsprechende Schritte einleiten kann, insbesondere in Bezug auf die Notwendigkeit der Bestrahlung durch die primäre und sekundäre Bestrahlungslichtquellen 32, 33, deren Intensität, oder auch in Bezug auf die jeweilige Bestrahlungsdauer.

Gemäß einer weiteren bevorzugten Ausführung, wie in Fig. 5 gezeigt, kann das erfindungsgemäße Handgerät auch in einer elektrisch betriebenen oder auch als automatisch wirkenden Zahnbürste 1' integriert werden. Die elektrische Zahnbürste 1' besteht dabei aus einem Zahnbürstenschaft oder Zahnbürstengrundkörper 2', einem austauschbaren Zahnbürstenkopf 3' mit einem Borstenträger 4' integriert. Eine Strom- bzw. Energiequelle 21' ist in dem Zahnbürstenschaft 2' integriert, beispielsweise in Form eines wiederaufladbaren Akkumulators, der kabellos geladen werden kann, wie zum Beispiel durch nicht drahtgebundene elektromagnetische Felder. Die elektrische Lichtzahnbürste 1' hat zudem wiederum einen Drucksensor (nicht gezeigt), der einen Anpressdruck des Borstenträgers 4' bzw. des Zahnbürstenkopfs 3' erfasst, wodurch erkennbar ist, ob sich der Borstenträger 4' bzw. der Zahnbürstenkopf 3' in Anpresskontakt mit einer Zahnoberfläche oder dergleichen im Mund- und Rachenraum befindet. In dem Zahnbürstenschaft 2' ist ferner eine Schaltungslogik 22' angeordnet, beispielsweise in der Form eines Computerchips oder dergleichen, der eine Stromverteilung an ebenfalls in dem Zahnbürstenschaft 2' angeordneten Lichtquellen 31', 32', 33' regelt, wobei zudem einen Schnittstelle in Gestalt eines Druckknopfs oder Schalters 23' in dem Zahnbürstenschaft 2' vorgesehen ist, durch welchen ein Anwender mit dem Computerchip interagieren kann, beispielsweise um die Ansteuerung der Lichtquellen 31', 32', 33' zu steuern. Der Schalter 23' kann dabei auch in Form eines Touchscreens vorgesehen sein, auf dem eine beispielsweise eine Energieversorgung der einzelnen Lichtquellen 31', 32', 33' angezeigt werden kann. Die Lichtquellen 31', 32', 33' befinden sich in dem Zahnbürstenkopf 3 der Zahnbürste 1' und umfassen bei dem vorliegenden Ausführungsbeispiel eine Anregungslichtquelle 31', eine primäre Bestrahlungslichtquelle 32' und eine optionale sekundäre Bestrahlungslichtquelle 33', die im Betrieb der Zahnbürste 1' in einer Anordnung von oben nach unten, bzw. in der Fig. 5 von rechts nach links angeordnet sind. Jede der Lichtquellen 31', 32', 33' besteht bei dem vorliegenden Ausführungsbeispiel aus einer LED bzw. einer Anordnung von mehreren LEDs, wie zum Beispiel ein sogenanntes LED-Array, wobei die LED-Lichtquellen 31', 32', 33' durch Betätigung des Schalters 23' angesteuert werden können. Ferner ist in dem vorliegenden Ausführungsbeispiel ein Motor 24' zum Betreiben einer Antriebswelle 241' vorgesehen, der ebenfalls durch die Energiequelle 21' betrieben und durch den Schalter 23' betätigt werden kann, wobei die Antriebswelle 241' zum Ausübung der mechanischen Bewegung des aufsteckbaren Bürstenkopfs 3' vorgesehen ist.

Wie es in Fig. 5 dargestellt ist, geben die LED-Lichtquellen 31, 32, 33 Strahlung ab, welche durch optische Fasern 311', 321', 331' zu dem Borstenträger 4' geführt und durch dessen Borsten 41' divergent hindurch nach außen abgestrahlt wird. Diese Strahlungsführung kann alternativ auch durch einen oder mehrere Spiegel als alternative Lichtführungswege ohne optische Lichtfasern umgesetzt werden. In dem vorliegenden Ausführungsbeispiel wird die Strahlenübertragung durch die Lichtfasern 311', 321', 331' umgesetzt, wobei das Lichtfaserbündel 311' die Anregungsstrahlung im Bereich von 400 nm bis 410 nm, vorzugsweise 405 nm, von der Anregungslichtquelle 31' nach durch die Borsten 41' hindurch leitet, das Lichtfaserbündel 321' die Bestrahlungsstrahlung im Bereich von 630 nm bis 700 nm, vorzugsweise 633 nm, von der primären Bestrahlungslichtquelle 32' nach außen durch die Borsten 41' hindurch leitet, und das optionale Lichtfaserbündel 331' die optionale Bestrahlungsstrahlung im Bereich von 490 nm bis 560 nm, vorzugsweise 505 nm, von der optionalen sekundären Bestrahlungslichtquelle 33' nach außen durch die Borsten 41' hindurch leitet, wobei eine Strahlungsführung von den Lichtquellen 31', 32', 33' nach außen entkoppelbar ist, um ein Austauschen des Bürstenkopfes 3' zu ermöglichen.

Die technischen Merkmale der vorhergehend beschriebenen Ausführungsformen sind nicht auf die jeweilige beschriebene Ausführungsform begrenzt und sind entsprechend untereinander austauschbar.

## Patentansprüche

1. Handgerät (1; 1') zur Anregung und Bestrahlung von pathogenen Mikroorganismen im Mund- und Rachenraum, mit
zumindest einer Anregungslichtquelle (31; 31') im kurzwelligen sichtbaren Spektralbereich zur Eigenfluoreszenzanregung und Bestrahlung der pathogenen Mikroorganismen an der Oberfläche, wobei die Anregungslichtquelle (31; 31') eine Anregungsstrahlung abgibt, welche einem Absorptionsmaximum von Porphyrinen entspricht,
zumindest einer primären Bestrahlungslichtquelle (32; 32') im roten Spektralbereich zur primären Bestrahlung der pathogenen Mikroorganismen und zur Transillumination, und
optional zumindest einer sekundären Bestrahlungslichtquelle (33; 33') im sichtbaren Spektralbereich von 450 nm bis 600 nm, wobei
die abgegebene Strahlung der Bestrahlungslichtquellen (32, 33; 32', 33') jeweils spektrale Anteile aufweist, welche von endogenen Porphyrinen, die durch die pathogenen Mikroorganismen produziert werden, absorbierbar sind, wodurch anhand von Folgeprozessen eine Fluoreszenzbildung und eine Inaktivierung der pathogenen Mikroorganismen eintritt, und
das Handgerät (1; 1') ferner einen Drucksensor aufweist, der bei Erfassung eines Andruckes des Handgeräts (1; 1') auf dem betroffenen Oberflächenbereich eine Bestrahlungslichtintensität auf Werte in einem Bereich von 10 mW/cm² bis 100 mW/cm² erhöht, um eine Inaktivierung der pathogenen Mikroorganismen zu erzielen.

2. Handgerät (1; 1') nach Anspruch 1, wobei die Anregungslichtquelle (31; 31') zur Eigenfluoreszenzanregung und Bestrahlung der pathogenen Mikroorganismen eine Anregungsstrahlung abgibt, welche in einem Bereich von 400 nm bis 410 nm liegt, vorzugsweise im Bereich um 405 nm.

3. Handgerät (1; 1') nach einem der vorangehenden Ansprüche, wobei die primäre Bestrahlung einen Wellenlängenanteil in einem Bereich von 630 nm bis 700 nm aufweist, vorzugsweise in einem Bereich von 630 nm bis 635 nm, weiter vorzugsweise im Bereich um 633 nm.

4. Handgerät (1; 1') nach einem der vorangehenden Ansprüche, wobei die optionale sekundäre Bestrahlung einen Wellenlängenanteil in einem Bereich von 490 nm bis 560 nm aufweist, vorzugsweise im Bereich um 505 nm.

5. Handgerät (1; 1') nach einem der vorangehenden Ansprüche, wobei das Abgeben der Strahlung der Anregungslichtquelle (31; 31'), der Strahlung der primären Bestrahlungslichtquelle (32; 32') und der optionalen Strahlung der sekundären Bestrahlungslichtquelle (33; 33') nach Art und Weise einer Ampelschaltung erfolgt.

6. Handgerät (1; 1') nach einem der vorangehenden Ansprüche, wobei das Handgerät (1; 1') ferner zumindest eine Lichterfassungseinrichtung zum Erfassen einer Eigenfluoreszenzstrahlung der pathogenen Mikroorganismen aufweist, vorzugsweise wobei das Handgerät (1; 1') zudem eine Sendeeinrichtung zum Übermitteln des Erfassungsergebnisses der Lichterfassungseinrichtung an eine externe Einrichtung aufweist.

7. Handgerät (1; 1') nach einem der vorangehenden Ansprüche, wobei die Anregungslichtquelle (31; 31'), die primäre Bestrahlungslichtquelle (32; 32') und/oder die optionale sekundäre Bestrahlungslichtquelle (33; 33') zumindest eine Licht-emittierende Diode LED, eine organische Leuchtdiode OLED und/oder einen Laser aufweist.

8. Handgerät (1; 1') nach einem der vorangehenden Ansprüche, wobei das Handgerät (1; 1') ein Zahnreinigungsgerät (1; 1') zum Zweck der Nutzung während der täglichen Dentalhygiene ist, vorzugsweise wobei sich die Anregungslichtquelle (31; 31'), die primäre Bestrahlungslichtquelle (32; 32') und/oder die optionale sekundäre Bestrahlungslichtquelle (33; 33') in einem Bürstenkopf (3; 3') des Zahnreinigungsgeräts (1; 1') befinden und eine Abstrahlung in Borstenrichtung erfolgt, weiter vorzugsweise wobei die Anregungslichtquelle bei geringer Intensität zur Bakterienerkennung im Rachenraum durch Fluoreszenzaktivierung von Porphyrinen in den pathogenen Mikroorganismen und bei höherer Intensität zur Inaktivierung oberflächlicher pathogener Mikroorganismen dient, die primäre Bestrahlungslichtquelle zur Inaktivierung tieferliegender pathogener Mikroorganismen und als Transmissionsquelle durch Transillumination zur Detektion und zur Verlaufsverfolgung von Sinusitis dient, und die optionale sekundäre Bestrahlungslichtquelle zur Inaktivierung tieferliegender pathogener Mikroorganismen dient.

9. Handgerät (1; 1') nach Anspruch 8, wobei
das Handgerät (1) eine manuell betriebene Zahnbürste (1) ist, vorzugsweise wobei der Bürstenkopf (3) mit einem auswechselbarem Borstenträger (4) versehen ist; oder
das Handgerät (1') eine elektrisch betriebene Zahnbürste (1') ist, vorzugsweise wobei der in Drehung und/oder Schwingung versetzbare Bürstenkopf (3') auswechselbar ist, und wobei eine Stromversorgung des elektrischen Antriebs (24') des Bürstenkopfs (3') und eine Stromversorgung der Anregungslichtquelle (31; 31'), der primären Bestrahlungslichtquelle (32; 32') und/oder der sekundären Bestrahlungslichtquelle (33; 33') durch dieselbe Energiequelle (21') erfolgt.

10. Handgerät (1; 1') nach einem der vorangehenden Ansprüche, wobei das Handgerät (1; 1') eine integrierte Energiequelle (21; 21') zur Stromversorgung der Anregungslichtquelle (31; 31'), der primären Bestrahlungslichtquelle (32; 32') und/oder der optionalen sekundären Bestrahlungslichtquelle (33; 33') aufweist, vorzugsweise wobei die integrierte Energiequelle (21; 21') kabellos aufladbar ist.

## Claims

1. A hand-held device (1; 1') for the stimulation and irradiation of pathogenic microorganisms in mouth and throat, comprising
at least one excitation light source (31; 31') in the short-wave visible spectral range for self-fluorescence excitation and irradiation of the pathogenic microorganisms at the surface, wherein the excitation light source (31; 31') emits an excitation radiation which corresponds to an absorption maximum of porphyrins,
at least one primary irradiation light source (32; 32') in the red spectral range for primary irradiation of the pathogenic microorganisms and for transillumination, and
optionally at least one secondary irradiation light source (33; 33') in the visible spectral range from 450 nm to 600 nm, wherein
the emitted radiation of the irradiation light sources (32, 33; 32', 33') has respective spectral components which are absorbable by endogenous porphyrins produced by the pathogenic microorganisms, whereby generation of fluorescence and inactivation of the pathogenic microorganisms occur by means of subsequent processes, and
the hand-held device (1; 1') further comprises a pressure sensor which, upon detecting a contact pressure of the hand-held device (1; 1') on the affected surface area, increases an irradiation light intensity to values in a range of 10 mW/cm² to 100 mW/cm² in order to effect inactivation of the pathogenic microorganisms.

2. The handheld device (1; 1') according to claim 1, wherein the excitation light source (31; 31') for self-fluorescence excitation and irradiation of the pathogenic microorganisms emits an excitation radiation which is in a range from 400 nm to 410 nm, preferably in the range around 405 nm.

3. The hand-held device (1; 1') according to any one of the preceding claims, wherein the primary irradiation has a wavelength component in a range from 630 nm to 700 nm, preferably in a range from 630 nm to 635 nm, further preferably in the range around 633 nm.

4. The hand-held device (1; 1') according to any one of the preceding claims, wherein the optional secondary irradiation has a wavelength component in a range from 490 nm to 560 nm, preferably in the range around 505 nm.

5. The hand-held device (1; 1') according to any one of the preceding claims, wherein the emission of the radiation of the excitation light source (31; 31'), the radiation of the primary irradiation light source (32; 32') and the optional radiation of the secondary irradiation light source (33; 33') is performed in the manner of traffic light-like indicators.

6. The hand-held device (1; 1') according to any one of the preceding claims, wherein the handheld device (1; 1') further comprises at least one light detection means for detecting an intrinsic fluorescence radiation of the pathogenic microorganisms, preferably wherein the handheld device (1; 1') further comprises a transmission means for transmitting the detection result of the light detection means to an external device.

7. The hand-held device (1; 1') according to any one of the preceding claims, wherein the excitation light source (31; 31'), the primary irradiation light source (32; 32') and/or the optional secondary irradiation light source (33; 33') comprise at least one of a light-emitting diode LED, an organic light-emitting diode OLED and/or a laser.

8. The hand-held device (1; 1') according to any of the preceding claims, wherein the hand-held device (1; 1') is a dental cleaning device (1; 1') for the purpose of use during daily dental hygiene, preferably wherein the excitation light source (31; 31'), the primary irradiation light source (32; 32') and/or the optional secondary irradiation light source (33; 33') are located in a brush head (3; 3') of the dental cleaning device (1; 1'), and an irradiation takes place in the bristle direction, further preferably wherein the excitation light source serves at low intensity for bacterial detection in the throat by fluorescence activation of porphyrins in the pathogenic microorganisms and at higher intensity for inactivation of superficial pathogenic microorganisms, the primary irradiation light source is for inactivation of deeper pathogenic microorganisms and as a transmission source by transillumination for detection and progression of sinusitis, and the optional secondary irradiation light source is for inactivation of deeper pathogenic microorganisms.

9. The hand-held device (1; 1') according to claim 8, wherein
the hand-held device (1) is a manually operated toothbrush (1), preferably wherein the brush head (3) is provided with a replaceable bristle carrier (4); or
the hand-held device (1') is an electrically operated toothbrush (1'), preferably wherein the brush head (3'), which can be set in rotation and/or oscillation, is exchangeable, and wherein a power supply of the electric drive (24') of the brush head (3') and a power supply of the excitation light source (31; 31'), the primary irradiation light source (32; 32') and/or the secondary irradiation light source (33; 33') is effected by the same energy source (21').

10. The hand-held device (1; 1') according to any one of the preceding claims, wherein the handheld device (1; 1') comprises an integrated power source (21; 21') for powering the excitation light source (31; 31'), the primary irradiation light source (32; 32') and/or the optional secondary irradiation light source (33; 33'), preferably wherein the integrated power source (21; 21') is wirelessly chargeable.

## Revendications

1. Appareil portatif (1 ; 1') pour l'excitation et l'irradiation de micro-organismes pathogènes dans la bouche et la gorge, avec
au moins une source de lumière d'excitation (31 ; 31') dans le domaine spectral visible à courtes longueurs d'ondes, pour l'excitation de la fluorescence propre et l'irradiation des micro-organismes pathogènes sur la surface, dans lequel la source de lumière d'excitation (31 ; 31') émet un rayonnement d'excitation qui correspond à un maximum d'absorption de porphyrines,
au moins une source de lumière d'irradiation primaire (32 ; 32') dans le domaine spectral rouge pour l'irradiation primaire des micro-organismes pathogènes et pour la transillumination et
en option au moins une source de lumière d'irradiation secondaire (33 ; 33') dans le domaine spectral visible de 450 nm à 600 nm, dans lequel
le rayonnement émis par les sources de lumière d'irradiation (32, 33 ; 32', 33') présente respectivement des parts spectrales qui peuvent être absorbées par des porphyrines endogènes, qui sont produites par les micro-organismes pathogènes, ce qui permet de provoquer, à l'aide de processus suivants, la formation d'une fluorescence et une inactivation des micro-organismes pathogènes et
l'appareil portatif (1 ; 1') comprend en outre un capteur de pression qui, lors de la détection de la pression de l'appareil portatif (1 ; 1') sur la zone de la surface concernée, augmente une intensité de lumière d'irradiation à des valeurs dans un intervalle de 10 mW/cm² à 100 mW/cm², afin d'obtenir une inactivation des micro-organismes pathogènes.

2. Appareil portatif (1 ; 1') selon la revendication 1, dans lequel la source de lumière d'excitation (31 ; 31') émet, pour l'excitation de fluorescence propre et l'irradiation des micro-organismes pathogènes, un rayonnement d'excitation qui se trouve dans un intervalle de 400 nm à 410 nm, de préférence dans l'intervalle autour de 405 nm.

3. Appareil portatif (1 ; 1') selon l'une des revendications précédentes, dans lequel l'irradiation primaire comprend une part de longueurs d'onde dans un intervalle de 630 nm à 700 nm, de préférence dans un intervalle de 630 nm à 635 nm, de préférence dans l'intervalle autour de 633 nm.

4. Appareil portatif (1 ; 1') selon l'une des revendications précédentes, dans lequel l'irradiation secondaire optionnel comprend une part de longueurs d'onde dans un intervalle de 490 nm à 560 nm, de préférence dans l'intervalle autour de 505 nm.

5. Appareil portatif (1 ; 1') selon l'une des revendications précédentes, dans lequel l'émission du rayonnement de la source de lumière d'excitation (31 ; 31'), du rayonnement de la source de lumière d'irradiation primaire (32 ; 32') et du rayonnement optionnel de la source de lumière d'irradiation secondaire (33 ; 33') a lieu à la manière d'un circuit à ampoules.

6. Appareil portatif (1 ; 1') selon l'une des revendications précédentes, dans lequel l'appareil portatif (1 ; 1') comprend en outre au moins un dispositif de détection de lumière pour la détection d'un rayonnement de fluorescence propre des micro-organismes pathogènes, de préférence dans lequel l'appareil portatif (1 ; 1') comprend en outre un dispositif émetteur pour la transmission du résultat de la détection du dispositif de détection de lumière à un dispositif externe.

7. Appareil portatif (1 ; 1') selon l'une des revendications précédentes, dans lequel la source de lumière d'excitation (31 ; 31'), la source de lumière d'irradiation primaire (32 ; 32') et/ou la source de lumière d'irradiation secondaire optionnelle (33 ; 33') comprend au moins une diode électroluminescente LED, une diode électroluminescente organique OLED et/ou un laser.

8. Appareil portatif (1 ; 1') selon l'une des revendications précédentes, dans lequel l'appareil portatif (1 ; 1') est un appareil de nettoyage des dents (1 ; 1') pour le nettoyage pendant l'hygiène dentaire quotidienne, de préférence dans lequel la source de lumière d'excitation (31 ; 31'), la source de lumière d'irradiation primaire (32 ; 32') et/ou la source de lumière d'irradiation secondaire optionnelle (33 ; 33') se trouvent dans une tête de brosse (3 ; 3') de l'appareil de nettoyage de dents (1 ; 1') et une irradiation a lieu dans la direction des poils, de préférence dans lequel la source de lumière d'irradiation permet, dans le cas d'une faible intensité, la reconnaissance des bactéries dans la gorge par activation de fluorescence de porphyrines dans les micro-organismes et, dans le cas d'un forte intensité, l'inactivation de micro-organismes pathogènes superficiels, la source de lumière d'irradiation primaire permet l'inactivation de micro-organismes pathogènes situés plus profondément et, en tant que source de transmission par transillumination, permet la détection et le suivi d'une sinusite et la source de lumière d'irradiation secondaire optionnelle permet l'inactivation de micro-organismes situés plus profondément.

9. Appareil portatif (1 ; 1') selon la revendication 8, dans lequel
l'appareil portatif (1) est une brosse à dents manuelle (1), de préférence dans lequel la tête de brosse (3) est munie d'un support de poils (4) interchangeable ; ou
l'appareil portatif (1') est une brosse à dents électrique (1'), de préférence dans lequel la tête de brosse (3'), qui peut être mise en rotation et/ou en oscillation, est interchangeable et dans lequel une alimentation électrique de l'entraînement électrique (24') de la tête de brosse (3') et une alimentation électrique de la source de lumière d'excitation (31 ; 31'), de la source de lumière d'irradiation primaire (32 ; 32') et/ou de la source de lumière d'irradiation secondaire (33 ; 33') a lieu par la même source d'énergie (21').

10. Appareil portatif (1 ; 1') selon l'une des revendications précédentes, dans lequel l'appareil portatif (1 ; 1') comprend une source d'énergie intégrée (21 ; 21') pour l'alimentation électrique de la source de lumière d'irradiation (31 ; 31'), de la source de lumière d'irradiation primaire (32 ; 32') et/ou de la source de lumière d'irradiation secondaire optionnelle (33 ; 33'), de préférence dans lequel la source d'énergie intégrée (21 ; 21') peut être rechargée sans fil.
